Europäisches Patentamt

European Patent Office

Office européen des brevets

(11) Publication number: **0 301 141**
**A1**

(12) # EUROPEAN PATENT APPLICATION

(21) Application number: 87306752.4

(22) Date of filing: 30.07.87

(51) Int. Cl.⁴: **G01N 33/543** , **G01N 33/544** ,
**//G01N33/569**

(43) Date of publication of application:
**01.02.89 Bulletin 89/05**

(84) Designated Contracting States:
**AT BE CH DE ES FR GB GR IT LI LU NL SE**

(71) Applicant: **MICROBIOLOGICAL RESEARCH
CORPORATION
40 West 500 South
Bountiful Utah 84010(US)**

(72) Inventor: **Golden, Carole Ann
3184 Wasatch Oaks Circle
Salt Lake City Utah 84124(US)**
Inventor: **Wilson, Albert Jerome
4600 South 2850 West, No. 195
West Valley City Utah 84119(US)**
Inventor: **Black, Melree K.
216 East 1950
South Bountiful Utah 84010(US)**

(74) Representative: **Ellis-Jones, Patrick George
Armine et al
J.A. KEMP & CO. 14 South Square Gray's Inn
London WC1R 5EU(GB)**

(54) **Solid phase enzyme immunoassay test apparatus and process for detecting antibodies.**

(57) A test apparatus and process for its use to simultaneously detect the presence of a variety of specific antibodies in a clinical specimen. The procedure involves an enzyme-linked dot blot immunoassay where an array of specific antigens that are used to detect specific antibodies are separately immobilized on a porous membrane (14) which is itself immobilized on a rigid support (11) to provide a dip stick (10). In the process specific antibodies are detected by sequential incubations of the antigen dip stick (10) in three different solutions of immunochemical reagents. The presence of specific antibody is evidenced by the appearance of a blue-violet color change to a dot of a specific antibody immobilized on the porous membrane (14) while the absence of color developement indicates the absence of such specific antibody.

FIG. 1

# SOLID PHASE ENZYME IMMUNOASSAY TEST APPARATUS AND PROCESS FOR DETECTING ANTIBODIES

## Field Of The Invention

The present invention relates to immunochemical systems and processes for determining the presence of antibodies or antigens in a clinical sample and relates most closely to a procedure known as enzyme-linked immunosorbent assay (ELISA).

## Prior Art

In the science of immunology, numerous procedures have been developed for disease detection, many of which are based on the understanding that an antigen is a substance that can induce a detectible immune response when introduced within an animal and an antibody is protein which is produced as a result of the introduction of that antigen and, in turn, that such antibody has the ability to combine with the antigen that stimulated its production. Therefore, a determination of the presence of a particular antibody or antigen can be determinative of whether the animal is suffering or has suffered from a particular disease or malady. With the identification of the presence of antibodies in a person's blood serum it can be concluded that an individual has been exposed to a particular type of antigen.

Tests for detection of antigens and antibodies in clinical practice have, in recent times, been broadly defined as occupying eleven categories: 1) immunodiffusion; 2) electrophoresis and immunoelectrophoresis; 3) immunochemical and physicochemical methods; 4) radioimmunoassay; 5) immunohisotchemical techniques; 6) agglutination; 7) complement fixation; 8) immunofluorescence; 9) precipitation; 10) viral neutralization, and 11) ELISA. With all of these techniques and procedures it is necessary to provide some visual record for analysis. An early approach to providing such a procedure and medium for reproducing data on a solid support was demonstrated by E.M. Southern in an article, "Detection of Specific Sequences Among DNA Fragments Separated by Gel Electrophoresis", Journal of Molecular Biology 98:503-517, 1975, wherein it was set out that DNA restriction fragments that had been fractionated according to size on an agarose gel could be transferred to a solid support and detected as discrete bands. This recognition provided the incentive for developing equivalent methods for detecting protein molecules in a complex mixture. One of the solid supports or porous membrane mentioned by Southern was nitrocellulose that is preferred as the support for the present invention. Of course, Southern was not the first use of nitrocellulose as prior thereto it had been in common use for a number of years as a support. Southern did, however, illustrate that it could be effectively used in a transfer of information from one medium to another. Now, numerous methods for transferring protein molecules to a variety of solid supports subsequent to their fractionation in a variety of gel matrices are currently avaiable. Some examples of generally known methods of protein transfer include capillary blotting, contact diffusion blotting and electroblotting that can involve nitrocellulose as a solid support.

Additional to membranes of nitrocellulose, there are three other principal types of immobilizing matrices that have been employed in protein blotting experiments. These include the diazo papers, cyanogen bromide activated papers, and nylon membranes. Two types of diazo papers are currently in use, diazobenzyloxymethyl (OBM) cellulose paper and diazophyenylthioether (DPT) cellulose paper. Where nitrocellulose papers have been found to be basically stable and have a good shelf life, the reactive diazo groups are unstable and have to be initially prepared in a stable intermediate form and are then chemically activated immediately prior to use. Diazo papers and cyanogen bromide activated papers require more preparation than the preferred nitrocellulose papers and must be used as soon as they are activated. While they have, however, the advantage of covalently binding of transferred proteins to the paper, though the precise mechanism of the reaction is not clear, the preparation they require makes them unsuitable for use with the present invention. Nylon membranes are stable and very durable, however, they have an extremely high affinity for protein binding. Because of this high affinity, immunochemical protein detection molecules tend to bind non-specifically to the membrane, lowering the level of sensitivity. Methods exist to minimize this non-specific binding including various blocking and washing strategies. Compared to nitrocellulose treatments, these blocking and washing strategies are, however, generally more rigorous and time consuming.

Membranes of nitrocellulose are currently the most widely used transfer medium for protein blotting and have the advantages of being relatively cheap, simple to use, and they also have a long shelf life of approximately 12 months at four degrees (4°) centigrade. Parallel blotting experiments have indicated that pure nitrocellulose filters, rather than mixed ester filters, provide for a more efficient binding of DNA. Therefrom it seems likely that this will also be the case for proteins. The precise nature of the protein nitrocellulose binding is not as yet fully understood but would seem to be the result of a number of different factors including hydrophobic and ionic interactions as well as hydrogen bonding. The capacity of the binding is determined by the available surface area of the filter which is inversely proportional to the pore size. It has been established that smaller proteins are more efficiently bonded with nitrocellulose when a 0.20 micron diameter pore size is used. Most workers prefer the 0.45 micron diameter pore filters for protein immobilization unless they are dealing with very small proteins. This information was used to construct the preferred porous membrane that with a backing as a support forms a "stick", of the invention, the nitrocellulose to receive dots of certain antigens and controls dried thereon.

As set out above, the present invention relates to a protein blotting system that is classified as an immunochemical technique and more specifically an enzyme-linked immunosorbent assay (ELISA). Over the past several years protein blotting to a solid support has been increasingly applied to solve a diverse range of problems. Such problems have involved the identification of DNA and RNA binding proteins, glycoproteins in addition to the screening of various antigens and antisera. Additionally, similar to the present invention, it has been common to utilize nitrocellulose membranes for protein blotting from fractionated proteins taken from gel matrices, and recent investigators have reported a use of this material for spotting protein samples directly thereon. This procedure is generally referred to as protein spotting or, as in the present invention, dot-immunobinding and is distinct from the earlier protein blotting from a gel matrices in that a measured aliquot of protein suspension is applied directly to the surface of the membrane and allowed to absorb into the membrane. Protein blotting from a gel matrices involves placing the fractionating gel matrices containing the immobilized proteins in direct contact with the nitrocellulose membrane and allowing transfer of proteins from the gel matrices to proceed by capillary action, contact-diffusion or electroblotting, all of which requires a great deal of time, employing several hours to several days.

The dot-immunobinding technique consists of applying a measured amount of protein solution onto the surface of the nitrocellulose membrane. Thereafter, such membrane is subjected to a blocking reaction where an agent is applied thereto to block protein binding for proteins other than those being tested for. After blocking, the spotted membrane will be used for detection of a specific antibody by subjecting the membrane to antigen-antibody reaction conditions. A bound specific antibody can be detected by application of either with [125] I labeled Staphylococcus aureus protein A or by using an appropriate anti-antibody labeled with [125] I, as set out in Burnett, W.N., 1981, "Western Blotting", Electrophoretic Transfer of Proteins from SDS-Polyacrylamide Gels to Unmodified Nitrocellulose and Radiographic Detection with Antibody and Radioiodinated Protein A, Anal. Biochem. 112: 195-203, with enzymes such as horseradish peroxidase or with fluorochromes such as fluorescein. Such bound radiolabeled conjugate is detected by autoradiography while the bound enzyme conjugate is detected by incubation in the presence of the appropriate chromagenic enzyme substrate, and fluorescein conjugates are detected by the presence of fluorescence as set out in Towbin, H., T. Stahelin and J. Gordon, "Electrophoretic Transfer of Proteins from Polyacrylamide Gels to Nitrocellulose Sheets," Proc. Natl. Acad. Sci. U.S.A. 76:4350-4354, 1979. Brooks, K.G., Shorma, S.P., and Remington, J.S., "Detection of Toxoplasma gondii Antigens By a Dot-Immunobinding Technique", Journal of Clinical Microbiology, 21:113-116, 1985, shows a use of horseradish peroxidase conjugated goat antirabbit to detect rabbit anti-Toxoplasma gondii antibodies bound to nitrocellulose-spotted T. gondii antigen, the article teaching the use of the peroxidase substrate hydrogen peroxide and 4-chloro-1-naphthal for color development.

The present invention is a new application of protein dot-immunobinding technique. It is unique in that it utilizes an immobilized nitrocellulose membrane in the form of a dip stick. It differs from other ELISA methods in that the colored products identifying a positive reaction are insoluble rather than soluble. This new application has the unique property of being able to test whole blood. No other serological procedure is currently performed on whole blood. Additionally, the present configuration allows for greater ease with which the immunochemical reactions may be executed including the handling of the membrane. The incubation temperatures employed are ambient as opposed to 37 degrees centigrade used for standard immunochemical assays. The sensitivity of the present configuration allows for very short incubation periods as opposed to the standard 30 minutes currently used. The present methodology is conducive to short water rinsing steps instead of the more time consuming rigorous rinsing conditions employed by current methodologies. Further, it is an improvment over prior systems in that it is both simple and reliable to use and is appropriate for use in the setting of a doctor's office to test in a single procedure for the presence of antibodies to a number of different antigens.

## SUMMARY OF THE INVENTION

It is a principal object of the present invention in a solid phase enzyme immunoassay test apparatus and process for detecting antibodies to provide an apparatus and procedure for accurately and reliably detecting the presence of a variety of specific antibodies in a clinical specimen in a relatively short period of time.

Another object of the present invention is to provide, as the test apparatus, a porous membrane immobilized on a rigid support whereto dots of specific antigens are separately immobilized that will, after sequential incubations in different immunochemical reagents, each individually react in the presence of a specific antibody by changing color to visually indicate the presence of such specific antibody.

Another object of the present invention is to provide a technique for immobilizing dots of specific antigens on a porous membrane to remain active over a significant period of time in ambient conditions.

Still another object of the present invention is to provide apparatus and procedure for simultaneously detecting, in a short period of time, specific antibodies in a clinical specimen in the setting of a medical doctor's office.

The present invention is an enzyme-linked dot blot immunoassay technique that simultaneously detects the presence of antibodies to several different antigens from a clinical specimen. The apparatus of the invention includes a porous membrane that is immobilized by attachment to one end of a support that is preferably formed from a rigid or semi-rigid plastic. Preferably, a tape having an adhesive on both faces is utilized to attach the porous membrane to the support, which support and tape, prior to receipt of the porous membrane, have ellipsoid holes formed at intervals therein that serve as windows exposing the porous membrane therethrough. An array of purified antigens are dot blotted onto the porous membrane in each of the separate windows or compartments. The stick mounting the immunodotted immobilized membrane is used in dip stick fashion for the detection of antibodies from clinical sample. To provide this detection, three five minute incubation steps are required. All of which incubation steps performed at ambient temperature. Three containers are provided with the invention for the immunochemcals in which the membrane bound end of the dip stick are sequentially inserted in the performance of the steps of the procedure.

The first container contains sample specimen diluent whereto a drop of either plasma, serum, or whole blood from a clinical specimen is added. The dip stick is inserted into the diluent and sample for a five minute incubation during which time antibodies from the clinical sample will bind to specific antigen spots. Following a brief rinse of the dip stick immunodotted immobilized membrane with distilled water the dip stick is inserted into a second container that preferably contains an alkaline phosphatase conjugated anti-human immunoglobulin G. During this incubation step enzyme conjugate binds to antibody from the clinical sample bound during the first step. Following another brief rinse the dip stick in the third step is inserted into the final container that preferably contains chromagenic substrates for the alkaline phosphatase. The alkaline phosphatase enzyme converts the soluble chromagenic substrates into colored insoluble products which bind to the porous membrane at the site of the bound enzyme conjugate. The bound products are thereby revealed as a colored spot on the membrane surface. The absence of a colored spot indicates the absence of antibody in the clinical sample. The formation of in insoluble product is unique in that current ELISA techniques for serology tests utilize an enzyme substrate that produces a soluble product.

In practice, the enzyme linked dot blot assay application in the present invention has been used to consistently and accurately identify the presence of antibodies to a variety of antigens. The present invention preferably provides an apparatus and procedure for simultaneously detecting antibodies to a number of different antigens.

## Brief Description of the Drawing

These and other objects and features of the present invention in a solid phase enzyme immunoassay test apparatus and process for its use for detecting antibodies to specific antigens in a clinical specimen will become more fully apparent from the following description in which the invention is described in detail in conjunction with the accompanying drawings.

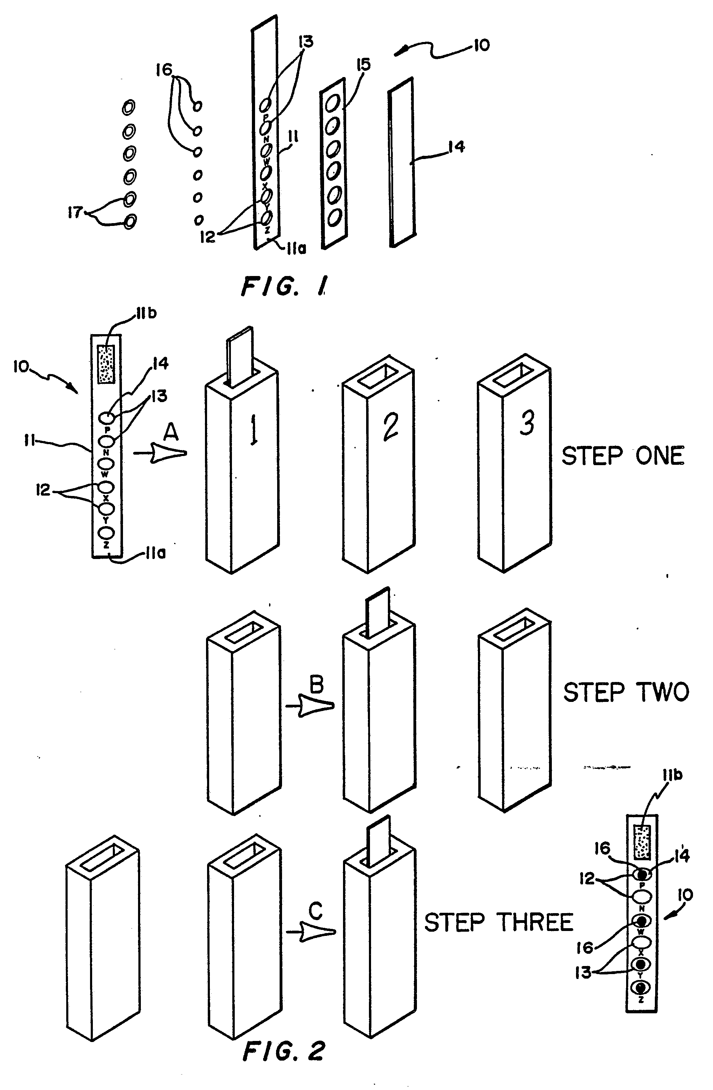

Fig. 1 is an exploded perspective view of stick support of the present invention shown as consisting of a solid support whereto an adhesive layer is positioned to be bonded, the support and adhesive layer to have ellipsoid holes punched at intervals therethrough with a porous membrane aligned to be fitted over the adhesive layer to receive, as separate dots, an array of purified antigens immobilized thereon; and

Fig. 2 is a flow schematic showing the steps involved in practice of the procedure of the present invention with the stick support of Fig. 1 for determining, by color change, the presence of antibodies to specific immobilized antigens in a clinical specimen.

## Detailed Description of Invention

In the present invention specific antibodies to several different antigens can be simultaneously detected from a single clinical specimen. Shown best in Fig. 1, the procedure preferably employs a dip stick 10 whereon are arranged an array of dot-immunobound purified antigens. The dip stick 10 includes a support 11 that is preferably formed from a long rectangular thin wafer of rigid plastic of approximately 25 mil thick with an array of ellipsoid windows 12 formed at intervals therein. The windows 12 are beveled inwardly at 13 at approximately fifteen degrees (15°) and are arranged at intervals from a lower end 11a past the dip stick middle. Of course, other types of plastic may also be employed as the support 11 and other window 12 designs are possible within the scope of this disclosure. A porous membrane 14 is attached to the dip stick 10 as by the application of a double back adhesive layer 15 to the support 11 that receives the porous membrane 14 on its other face. Which adhesive layer could also be a liquid layer, or the like, and where a double back adhesive layer is used that layer is holed strategically to conform to the windows in the support 11, exposing a section of the layer of porous membrane 14 through each window 12. In practice, a nitrocellulose membrane layer is preferred as the porous membrane. Though, it should be understood, other porous membranes may also be used to include nylon or other affinity membranes within the scope of this disclosure. Embossed characters such as P, N, W, X, Y, and Z, as shown in Figs. 1 and 2, are preferably formed on the support 11 near each window 12 for identifying each window. A roughened surface is provided on a handle end 11b, shown best in Fig. 2, of the dip stick 10 to provide for indicating clinical sample identification thereon.

Illustrated in Fig. 1, once a dip stick 10 has been assembled, the adhesive layer bonding the porous membrane to the support, the porous membrane in the individual windows 12 are spotted (immunodotted) with one microliter each of various antigen samples, shown as dots 16. Though volumes other than one microliter may also be employed. The different samples are dispensed as dots 16 into the center of each ellipsoid windows W, X, Y, and Z. One of the windows 12, a second window from the top labeled N, is reserved for a negative control. Such control as is spotted in this window 10 consists of a buffer solution used for the solubilization/dilution of all the other samples to be spotted in the remaining windows or can be a negative control antigen such as a cellular extract of uninfected cells used for viral antigen production. Further, a separate window identified as P, that is preferably the top window adjacent to end 11b allocated for a positive control. The positive control is preferably spotted in the top window P. This control is used to verify that all reagents are functioning properly and that the test procedures have been properly executed. The control also verifies that the three reaction vessels are sufficiently reconstituted to the desired volume to successfully perform the test procedure. This window is spotted with a solution of purified human immunoglobulin G (IgG). Of course, solutions other than those described may be used as the positive and negative controls or may be omitted entirely and windows other than those designated as P and N could be used within the scope of this disclosure. The remaining windows 12 are each to be spotted with various purified antigens that are each specific for a type of antibody in question. The total number of types of antibodies under consideration determines the number of ellipsoid windows employed. In practice, as set out in the examples below, a dip stick incorporating six windows, two for, respectively, the positive and negative controls with the other four windows spotted with various purified antigens has been used successfully.

Once the porous membrane 14 within windows 12 has been spotted with the appropriate solutions the spots 16 are allowed to air dry at ambient temperature. Drying time usually requires several minutes though very long drying times may be employed without noticeable effect. Mild temperatures other than ambient may also be employed without noticeable effect.

Following drying, the nitrocellulose membrane-bound end 11a of the dip stick 10 is blocked or capped, as illustrated by the centrally holed ellipsoid shaped disks 17 that represent coating of the membrane. Blocking is employed to occupy the nitrocellulose with a non-interfering protein, or other like compound that will prevent the non-specific binding of the immunochemical compounds thereto, the blocking minimally effecting the spots 16. Blocking is preferred due to the high affinity of most porous membranes, such as nitrocellulose, to non-specific adsorption of a wide range of proteins. In the present invention, to provide this blocking, the membrane-bound end 11a of the dip stick 10 is immersed in a solution containing non-fat dry milk in a Tris-saline solution (5% non-fat dry milk in 10 mM Tris, pH 7.4, 0.9% NaCl) for 15 minutes at ambient temperature. Other blocking solutions may also be employed to include solutions of albumin, caesin, gelatin, detergents, amino rich compounds, and others, and the time and temperature of the blocking may be varied depending on the membrane employed and the desired level of acceptable sensitivity/interference. Blocking may be omitted entirely for some applications where lower level of sensitivity is acceptable. Following blocking, dip stick 10 is briefly rinsed in distilled water and allowed to air dry at ambient temperature. Other rinse solutions may also be employed such as buffered or detergent containing solutions where greater levels of sensitivity for a particular test configuration are desired. The blocked dip sticks 10 can then be packaged in foil pouches and stored at refrigerator temperature until use, or they may be packaged in other suitable containers or not packaged at all depending on their anticipated use. Packaged or unpackaged dip sticks 10 may also be stored at ambient temperature for some applications. Further, while not shown, two such dip sticks 10 could be manufactured or arranged together in back to back configuration to double the number of specific antibodies that can be tested for in a single procedure.

Fig. 2 illustrates a practice of the process of the present invention as including sequential incubation steps of the dip stick 10 of Fig. 1 in containers 1, 2 and 3. As illustrated therein, to detect a specific antibody from a clinical sample a prepared dip stick 10, prepared as set out above, is sequentially incubated at ambient temperature for five (5) minutes in three separate immunochemical solutions contained, respectively, in containers 1, 2 and 3 with brief rinsing of the dip stick in distilled water between each incubation. While the steps are preferably performed under ambient conditions other incubation temperatures and times may also be employed to effect a speed or slow down of each incubation within the scope of this disclosure.

The process is preferably performed in small plastic containers, one for each immunochemical solution, arranged to receive the nitrocellulose bound membrane end dip stick 10. Each of the containers 1, 2 and 3 contain dry immunochemical reagents, that are reconstituted with distilled water. Prior to dip stick membrane end immersion, a disposable plastic pipette, not shown, is used to insure adequate mixing of the resuspended reagents by forcing the liquid contents of each container up and down several times. Additionally, non-lyophilized reagents may also be employed where it is desired to utilize liquid reagents in concentrated form, distilled water is added to effect the proper desired dilution or, as needed, already diluted reagents may be added to the containers.

Shown in Fig. 2, the first of the three containers, numbered 1, contain s a clinical sample diluent that is provided to dilute the clinical sample to a less concentrated form and also provides blocking agents to minimize non-specific binding of the clinical sample to the membrane. A preferred reconstituted diluent contains 5% non-fat dry milk in 10mM Tris, pH 7.4, 0.9% NaCl, 0.01% thimerosal (TSM). Additionally, within the scope of this disclosure, other solutions may also be employed as diluent such as, for example, Tris-saline, phosphate buffered saline, detergent containing solutions, and the like, so long as such solution minimizes non-specific interactions of the immunochemical reagents. After reconstitution, a drop of either serum, plasma, or whole blood is added to container 1 and adequate mixing is again insured by using a disposable pipette, not shown. In practice, it has been found that a very small or very large drop of blood or several drops of blood may be added to the sample diluent without affecting test results. (An average drop is approximately 45-50 microliters). Where several drops of whole blood are desired for a particular application, coagulation problems may be avoided by employing the diluted blood sample with relative haste such that the sample will have been used and discarded prior to the onset of coagulation. Alternatively, an anti-coagulant may be included as part of the lyophilized reagent or added to the resuspended diluent. During this first incubation step, if present, a specific antibody from the clinical sample will bind to the spot of a specific antigen.

The above is set out in Fig. 2 as step 1, the dip stick 10, incubated in the presence of the clinical sample in the sample diluent in container 1, illustrated as arrow A. After incubation the dip stick is removed from the container 1 and is briefly rinsed with distilled water, illustrated as arrow B in step 2. In this step other solutions than distilled water may be used for the rinse (and all subsequent rinse steps) to provide an acceptable level of sensitivity/interference of the now activated dots 16.

Following the rinse, arrow B in step 2, the dip stick 10 is inserted in container 2 that preferably contains a gamma chain-specific enzyme conjugated goat anti-human IgG, although conjugates of other specificities are also within the scope of the present invention. This use of an enzyme conjugate is essentially like other enzyme-linked immunosorbent assay (ELISA) based procedures where enzyme conjugates rather than fluorescent labeled conjugates are employed to provide greater sensitivity. The reconstituted reagent used in container 2 preferably consists of alkaline phosphatase conjugate diluted in a protein-containing high salt stabilization buffer (1% bovine serum albumin in 50 mM Tris, 1 mM $MgCl_2$, 1M NaCl, 0.01% thimerosal, pH 7.5). The high salt concentration to minimize non-specific reactions. Additionally, other stabilization buffers may be employed in this reagent depending on the conjugate used and the desired level of sensitivity and the requirement for non-interference from non-specific binding. Other enzyme-conjugates such as horseradish peroxidase, beta-galactosidase, urease, and the like, may also be employed so long as, during the incubation of the dip stick 10 in container 2, the enzyme labeled conjugate binds to clinical sample antibodies that have bound to specific antigen spots from the first incubation in container 1. Additionally, the conjugate will also bind to the positive control spot (human IgG) during this incubation.

Following a brief rinsing in distilled water, illustrated as arrow C in step 3, the dip stick 10 is incubated in container 3. This third container contains enzyme substrates for the enzyme conjugate which react with the conjugate in such a way as to reveal the presence of said conjugate in a visual way. When reconstituted a preferred solution will contain 0.404 mM nitro blue tetrazolium (NBT), 0.384 mM 5- bromo-4-chloro-3-indolyl phosphate (BCIP), 100 mM Tris, pH 9.5, 100 mM NaCl, and 50 mM $MgCl_2$. Other enzyme substrates may be substituted for the NBT/BCIP within the scope of this disclosure so long as such would produce a visible color change to the particular dot 16. During this incubation period the preferred alkaline phosphatase interacts with the NBT/BCIP substrates. Shown as the product of step 3, a result of this enzymatic interaction is the production and deposition of insoluble colored precipitates on the nitrocellulose membrane 14 at the site of the bound enzyme conjugate dot 16. This deposition and its tight binding to the membrane produces on the dip stick 10 a blue-violet colored spot 16 against a white background of the nitrocellulose membrane 14 within the ellipsoid window 12. Following a brief rinse the dip stick is examined for the presence or absence of the blue-violet color within each of the individual ellipsoid windows. Presence of colored spots within a window indicates the presence of a specific antibody in the clinical specimen for that specific antigen while the absence of a colored spot indicates the absence of such specific antibody. Further, as a verification of the validity of the test results, examination of the positive and negative control windows P and N should indicate a colored spot in the positive control window and no detectable spot in the negative control window. Results other than those indicated for the two controls invalidates the test. This is because a color change of the positive control and no color change of the negative control indicates that all test reagents are working properly at the time of testing and that the proper procedures for executing the test have been followed.


Example

In practice, the enzyme-linked dot blot immunoassay, hereinafter referred to as dot blot, is to identify antibodies from clinical specimens to a wide variety of antigens including Toxoplasma gondii (Toxo), Epstein Barr virus (EBV), Cytomegalovirus (CMV), Herpes simplex virus (HSV), Rubella virus, Chlamydia trachomatis, Human T cell lymphotropic virus type III (HTLV III), and others. In one clinical evaluation, the simultaneous identification of antibodies to Toxo, EBV, CMV and HSV were investigated, each test utilizing a single treated dip stick. Test results from clinical specimens obtained by the dot blot methodology were compared to indirect immunofluorescent antibody (IFA) test results. The IFA methodology is currently a Food and Drug Administration (FDA) approved methodology for testing for the presence of antibodies to these four antigens. For this test both serum and whole blood fractions were obtained from one hundred individuals chosen at random from the clinical study. Aliquots of the serum samples were tested by the approved IFA procedure as well as the dot blot system of the present invention. Four separate IFA tests were required to test each serum from each of the 100 clinical specimens for the four different antigens. A single dip stick test was performed on each of the 100 sera, each test strip detecting the presence of antibody to the four antigens simultaneously. The results of this comparison are shown in Table I. Compared to IFA, the percent specificity for the dot blot system of the present invention for the four antigens ranged from 96.3% for HSV antibody to 100% for the other three antigens or an average of 99.08% for the combined four. Percent sensitivity ranged from 90.9% for EBV to 100% for Toxo or a combined average for the four of 95.65%. Percent reproducibility ranged from 95% for EBV to 100% for CMV with a combined average for the four of 97.5%.

Equally impressive is the comparison of whole blood test results as shown in Table 2. The percent sensitivity to IFA test results ranged from 92.1% for EBV to 100% for Toxo with an average of 96.4% for the four. The percent specificity ranged from 96.3% for HSV to 100% for Toxo and EBV with a combined average of 98.2% for the four antigens. The percent reproducibility ranged from 95% for EBV to 100% for CMV with an average of 97.5% for the four.

7

TABLE I*

| Antigen | %Sen | %Spe | %Rep | %F+ | %F- | T % Error |
|---|---|---|---|---|---|---|
| Toxoplasma gondii | 100 | 100 | 96.7 | 0 | 6.7 | 3.3 |
| Epstein Barr virus | 90.9 | 100 | 95 | 6.7 | 3.3 | 5 |
| Cytomegalovirus | 94.4 | 100 | 100 | 0 | 0 | 0 |
| Herpes simplex virus | 97.3 | 96.3 | 98.3 | 3.3 | 0 | 1.7 |

* Note - Comparison of the dot blot system results of sera of 100 clinical specimens with Indirect Immunofluorescent Antibody (IFA) test results. %Sen = % sensitivity, %Spe = % specificity, %Rep = % reproducibility, %F+ = % false positives, %F- = % false negatives, and T%Error = total % error. For reproducibility testing, a minimum of 20 of the clinical samples were retested on three different days using the dot blot system of the present invention.

TABLE II*

| Antigen | %Sen | %Spe | %Rep | %F+ | %F- | T % Error |
|---|---|---|---|---|---|---|
| Toxoplasma gondii | 100 | 100 | 96.7 | 0 | 6.7 | 3.3 |
| Epstein Barr virus | 92.1 | 100 | 95 | 6.7 | 3.3 | 5 |
| Cytomegalovirus | 97.4 | 96.6 | 100 | 0 | 0 | 0 |
| Herpes simplex virus | 95.9 | 96.3 | 98.3 | 0 | 3.3 | 1.7 |

* Note - Comparison of dot blot system results of whole blood of 100 clinical specimens with Indirect Immunofluorescent Antibody (IFA) test results. %Sen = % sensitivity, %Spe = % specificity, %Rep = % reproducibility, %F+ = % false positives, %F- = % false negatives, and T%Error = total % error . For reproducibility testing, a minimum of 20 of the clinical samples were retested on three different days using the dot blot of the present invention.

As can be seen by the data in Tables 1 and 2, there is an extremely high correlation between dot blot system results of the present invention and IFA test results. The high correlation is evidenced by high percentages that are all within the 90 - 100% range. It is of particular interest to note extremely close correlation of whole blood test results to serum test results using the methodology of the present invention. This is of particular significance since whole blood is not presently used in serological testing. Cellular components of whole blood cause non-specific reactions in serological procedures greatly reducing specificity and sensitivity. This interference is totally absent in the dot blot system of the present invention as evidenced by the close correlation of serum test results with whole blood test results. This is of tremendous advantage because a single drop of whole blood obtained from a finger prick can be used in the test where this is not possible with other serological procedures. Routine serological tests require that a larger volume of blood be obtained from the patient by venapuncture by a skilled technician. Furthermore, the whole blood sample, for other testing methods has to be processed by centrifugation to obtain the serum fraction prior to testing.

Additionally, the dot blot system of the present invention for testing for the presence of antibody has been found in practice to have several advantages over standard serological procedures. The test can identify the presence of antibody to several antigens simultaneously. The test can be performed using a single drop of whole blood. Performance of the test does not require highly trained personnel and sophisticated instrumentation. The test can be done very rapidly, in less than 20 minutes. The test has its own built in positive and negative controls. The test is easily read and the test dip stick can be kept as a permanent record.

While a preferred embodiment of the invention in a test apparatus for detecting antibodies in a clinical specimen and process for its use have been shown and described herein, it should be apparent that this disclosure is made by way of example only and that variations to the apparatus and process are possible within the scope of this disclosure without departing from the subject matter coming within the scope of the following claims, which claims we regard as our invention.

## Claims

1. A solid phase enzyme immunoassay system for visually detecting specific antibody presence in a clinical sample comprising a chemically neutral backing member (11) having a plurality of openings (12) therethrough, a porous membrane (14) for receiving and maintaining the individual integrity of dots of selected purified antigens blotted at intervals thereon, bonding means (15) for immobilizing said porous membrane (14) on said backing member (11) and to expose sections of said porous membrane (14) through said backing member openings (12), blotting individual dots of select purified antigens on said porous membrane (14) one in each said opening (12), a vessel for sequentially receiving and incubating said backing member (11) mounting said porous membrane (14) in immunochemical solutions contained in said vessel including a clinical sample diluent whereto an amount of plasma serum or whole blood of approximately one drop is added, an enzyme conjugate that will bind to a specific antibody as may be present in the clinical sample and a specified purified antigen dot, and an enzyme conjugate substrate to interact with the enzyme conjugate that binds said specific antibody to said dot of specified purified antigen to product a visual color change.

2. An immunoassay system according to Claim 1, wherein the backing member (11) is formed from a rigid or semi-rigid plastic.

3. An immunoassay system according to Claim 1 or 2, wherein the porous membrane (14) is formed from nitrocellulose.

4. An immunoassay system according to Claim 1, 2 or 3, wherein the openings (12) are ellipsoidal windows.

5. An immunoassay system according to Claim 4, wherein the windows are beveled inwardly at approximately a fifteen degree (15°) angle.

6. An immunoassay system according to any one of the preceding claims, further including markings on the backing member (11), one proximate to each opening (12) for identifying a specific purified antigen dot immobilized on the porous membrane (14) in that opening (12), and the openings (12) are formed at spaced intervals toward a lower end (11a) to leave at the other end a finger engaging portion.

7. An immunoassay system according to any one of the preceding claims wherein the bonding means (15) is a layer of double backed adhesive fitted to the backing member (11) and has openings therethrough that conform to the openings (12) in said backing member (11), the porous membrane (14) fitted thereover sandwiching the adhesive between it and the backing member (11) so that a section of that porous membrane (14) is displayed in each said opening.

8. An immunoassay system according to any one of the preceding claims wherein six openings (12) are formed at spaced apart intervals in said backing member (11) each to expose a section of porous membrane (14) therein, four openings to each receive a specific purified antigen dot immobilized on said section of porous membrane (14) therein, the other two openings receiving, respectively, a dot each of positive and negative controls immobilized thereon.

9. An immunoassay system according to Claim 8, wherein the positive control is a dot of purified human immunoglobulin G (IgG) that will exhibit a color change after the sequential incubations in the immunochemical solutions that is indicative that the test reagents are working properly and that proper test procedures have been followed; and the negative control is either a dot of a buffer solution appropriate for the solubilization/dilution of the other antigen dots spotted on the other windows or a negative control antigen such as a cellular extract of uninfected cells used for viral antigen production, the negative control color to remain unchanged with the sequential incubations in said immunochemical solutions if the test reagents are working properly and the proper test procedures have been followed.

10. An immunoassay system according to any one of the preceding claims, wherein the area of the porous membrane section in each opening (12) is blocked with a non-interfering protein for preventing non-specific binding of immunochemical reagents.

11. An immunoassay system according to Claim 10, wherein the non-interfering protein is a solution containing non-fat dry milk in a Tris-saline solution.

12. An immunoassay system according to Claim 10 or 11 wherein the porous membrane is dotted with purified antigens, allowed to air dry, the blocking non-interfering protein is applied as a solution, the porous membrane dipped therein, and following a brief rinse is allowed to air dry.

13. An immunoassay system according to any one of the preceding claims wherein the sample diluent is a buffer solution selected to minimize non-specific interactions of the immunochemical reagents.

14. An immunoassay system according to any one of the preceding claims, wherein the enzyme conjugate is one selected to minimize non-specific interactions.

15. An immunoassay system according to Claim 14, wherein the enzyme conjugate is an alkaline phosphatase conjugate that has been diluted in a protein-containing high salt stabilization buffer solution.

16. An immunoassay system according to any one of the preceding claims, wherein the enzyme conjugate substrate is one that will react to the selected enzyme conjugate to produce a detectable color change at the antigen dot.

17. An immunoassay system according to any one of the preceding claims, wherein the dotted exposed sections of porous membrane between incubations are rinsed with a wash solution that minimizes non-specific binding of the immunochemicals, which wash solution will not interfere with the specific interactions of said immunochemicals.

18. An immunoassay system according to Claim 17, wherein the wash solution is distilled water.

19. A process for visually detecting specific antibody presence in a clinical sample comprising the steps of:
immobilizing dots of specific purified antigen blotted at intervals on a porous membrane that is backed with a backing member; successive incubations of the porous membrane mounting the antigen dots in select immunochemicals, a first incubation of said dots in a sample diluent consisting of buffers whereto has been added a small amount of plasma, serum or whole blood of approximately one drop forming a clinical sample, the diluent selected to dilute the sample while minimizing non-specific immunochemical interactions does not interfere with desired chemical reactions; a second incubation of said antigen dots in an enzyme conjugate that is suitable for performing an enzyme-linked immunosorbent assay (ELISA) based procedure where said conjugate will bind to the clinical sample antibodies that have bound to certain of said specific antigen spots on the porous membrane; a third incubation of said dots in an enzyme conjugate substrate of the enzyme of the second incubation selected to produce a detectable dot color change; and washing the porous membrane at the antigen dots between incubations with a wash solution selected to minimize non-specific binding of immunochemicals that does not interfere with the specific interactions of said immunochemicals.

20. A process according to claim 19, when carried out using an immunoassay system as defined in any one of claims 1 to 18.

FIG. 1

STEP ONE

STEP TWO

STEP THREE

FIG. 2

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | JOURNAL OF CLINICAL MICROBIOLOGY, vol. 21, no. 1, January 1985, pages 113-116, American Society for Microbiology; R.G. BROOKS et al.: "Detection of Toxoplasma gondii antigens by a dot-immunobinding technique" * Pages 113-114 * | 1-5,7, 10,12, 16,17, 19,20 | G 01 N 33/543 G 01 N 33/544// G 01 N 33/569 |
| Y | FR-A-2 581 194 (LE MATERIEL BIOMEDICAL) * Whole document * | 1-5,7, 10,12, 16,17, 19,20 | |
| Y | DE-A-2 541 031 (R. FUHR) * Whole document * | 1-5,7, 10,12, 16,17, 19,20 | |
| A | EP-A-0 174 247 (B. GUERIN et al.) * Whole document * | 1-3,7- 10,12- 14,16- 20 | |
| A | US-A-4 059 407 (H.T. HOCHSTRASSER) * Figures; column 13, lines 19-45 * | 1-20 | **TECHNICAL FIELDS SEARCHED (Int. Cl.4)** G 01 N |
| A | GB-A-2 180 645 (ENVIRONMENTAL DIAGNOSTICS INC.) * Figures; page 1, line 106 - page 3, line 130 * | 1-20 | |
| A | ANALYTICAL BIOCHEMISTRY, vol. 119, 1982, pages 142-147, Academic Press, Inc.; R. HAWKES et al.: "A dot-immunobinding assay for monoclonal and other antibodies" * Whole document * ---            -/- | 1 | |

The present search report has been drawn up for all claims

| Place of search | Date of completion of the search | Examiner |
|---|---|---|
| THE HAGUE | 05-04-1988 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 558 013  (V.A. MARINKOVICH) | | |
| A | EP-A-0 063 810  (CIBA-GEIGY AG) | | |

TECHNICAL FIELDS SEARCHED (Int. Cl.4)

The present search report has been drawn up for all claims

| Place of search | Date of completion of the search | Examiner |
|---|---|---|
| THE HAGUE | 05-04-1988 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention ·
E : earlier patent document, but published on, or after the filing date
D : document cited in the application
L : document cited for other reasons

 

& : member of the same patent family, corresponding document

EPO FORM 1503 03.82 (P0401)